# FASCICULE DE BREVET EUROPEEN

(11) **EP 2 744 543 B1**
(45) Date de publication et mention de la délivrance du brevet: **07.10.2015**
(21) Numéro de dépôt: 12759797.9
(22) Date de dépôt: 13.08.2012
(51) Int. Cl.: A61M 15/00

(54) **DISPOSITIF D'INHALATION DE POUDRE**
PULVERINHALATOR
POWDER INHALING DEVICE

(30) Priorité: 19.08.2011 FR 1157414
(43) Date de publication de la demande: 25.06.2014
(73) Titulaire: Aptar France SAS, 27110 Le Neubourg (FR)
(72) Inventeur: COLOMB, Arnaud, 78480 Verneuil sur Seine (FR)
(74) Mandataire: CAPRI
(86) Numéro de dépôt international: PCT/FR2012/051884
(87) Numéro de publication internationale: WO 2013/026977

(56) Documents cités:
- WO-A1-01/56640
- WO-A1-90/15635
- WO-A1-2010/004229

## Description

La présente invention concerne un dispositif d'inhalation de poudre, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés du type blister, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. Ceci rend toutefois les dispositifs complexes et donc coûteux à fabriquer et à assembler. Le document WO 2010/004229 décrit un inhalateur comportant une chambre de dispersion, le flux d'air chargé de poudre entrant dans ladite chambre de dispersion par une entrée tangentielle et sortant de ladite chambre de dispersion par une sortie perpendiculaire à ladite entrée tangentielle.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, avec une bonne précision de dosage et une reproductibilité du dosage à chaque actionnement.

La présente invention a donc pour objet un dispositif d'inhalation de poudre, comportant un corps pourvu d'un orifice de distribution, une pluralité de réservoirs prédosés contenant chacun une dose de poudre à distribuer, et des moyens d'ouverture de réservoir pour ouvrir un réservoir à chaque actionnement, le dispositif comportant une chambre de dispersion comportant une entrée reliée lors de l'inhalation à un réservoir ouvert et recevant le flux d'air et de poudre à partir dudit réservoir ouvert via un canal d'amenée, et une sortie reliée audit orifice de distribution via un canal de distribution, ladite chambre de dispersion comportant au moins une bille déplaçable sur un chemin de bille dans ladite chambre de dispersion, ladite chambre de dispersion comportant au moins une entrée d'air environ tangentielle par rapport audit chemin de bille, caractérisé en ce que lesdits canaux d'amenée et de distribution s'étendent dans une même direction sensiblement perpendiculaire audit chemin de bille et à ladite au moins une entré d'air tangentielle.

Selon une première variante avantageuse, ledit canal d'amenée est sensiblement coaxial avec ledit canal de distribution, lesdites entrée et sortie de la chambre de dispersion étant disposées sensiblement au centre de ladite chambre de dispersion.

Avantageusement, ladite entrée de la chambre de dispersion comporte un déflecteur pour dévier le flux d'air et de poudre vers le chemin de bille de ladite chambre de dispersion.

Avantageusement, ledit déflecteur est en forme de coupelle avec le sommet extérieur de ladite coupelle tourné face au flux d'air et de poudre entrant.

Selon une seconde variante avantageuse, ledit canal d'amenée est décalé par rapport audit canal de distribution, ladite entrée de la chambre de dispersion étant disposée au niveau du chemin de bille et ladite sortie de la chambre de dispersion étant sensiblement au centre de ladite chambre de dispersion.

Avantageusement, ladite entrée de la chambre de dispersion comporte une grille.

Avantageusement, chaque entré d'air de la chambre de dispersion est formée par un canal d'air tangentiel.

Avantageusement, ladite pluralité de réservoirs est formée sur une bande de blisters souple allongée, comportant une couche de base contenant les cavités des réservoirs et une couche de fermeture recouvrant lesdites cavités.

Avantageusement, la couche de fermeture est pelable de la couche de base, la partie de couche de base contenant les blisters vides s'enroulant autour d'un premier élément de réception rotatif et la partie de couche de fermeture pelée de ladite couche de base s'enroulant autour d'un second élément de réception rotatif.

Avantageusement, lesdits moyens d'ouverture de réservoirs comportent des moyens pour décoller ladite couche de fermeture de ladite couche de base, tels qu'un bord de pelage autour duquel s'étend ladite couche de fermeture, le dispositif comportant des moyens de déplacement, tel qu'une roue d'indexage, pour faire avancer la bande de blisters avant et/ou pendant chaque actionnement, l'extrémité aval de ladite couche de fermeture étant fixée à un second élément de réception rotatif dont la rotation est corrélée à la rotation de ladite roue d'indexage.

Avantageusement, ladite chambre de dispersion contient une pluralité de billes.

Avantageusement, après ouverture, ledit réservoir ouvert se trouve disposé face d'une part audit canal d'amenée et face à un canal d'arrivée d'air, de sorte que lors de l'inhalation, un flux d'air va pénétrer dans ledit blister à travers ledit canal d'arrivée d'air et entraîner ladite poudre dans un flux d'air et de poudre via ledit canal d'amenée vers ladite chambre de dispersion.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un dispositif d'inhalation de poudre selon un premier mode de réalisation avantageux de l'invention,
- la figure 2 est une vue de détail en section transversale d'une partie du dispositif de la figure 1,
- la figure 3 représente une vue schématique en perspective découpée de la partie du dispositif d'inhalation représentée sur la figure 2,
- la figure 4 est une vue schématique en section horizontale de la chambre de dispersion,
- la figure 5 est une vue similaire à celle de la figure 2, montrant partiellement un second mode de réalisation avantageux de l'invention,
- la figure 6 est une vue en perspective du dispositif avec les parties de capot ouvertes,
- la figure 7 est une vue similaire à celle de la figure 3, montrant le second mode de réalisation, et
- la figure 8 est une vue similaire à celle de la figure 4.

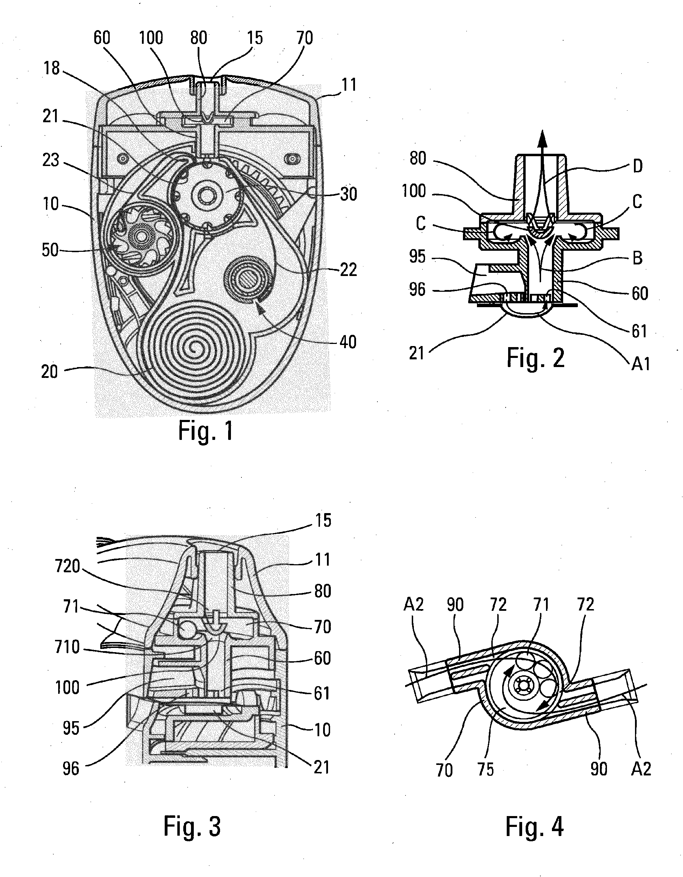

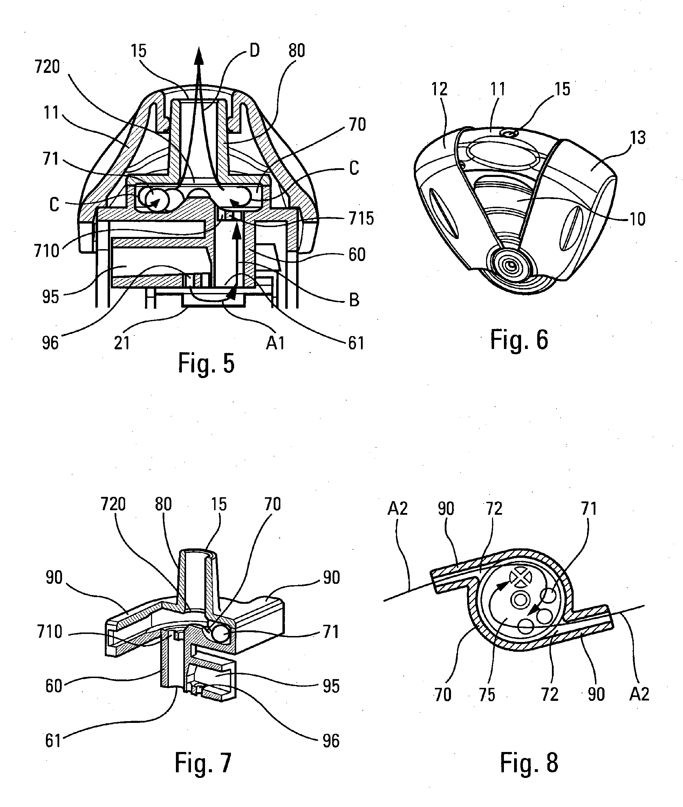

Sur la figure 1 est représentée une variante de réalisation avantageuse d'un inhalateur de poudre sèche. Cet inhalateur comporte un corps 10, sur lequel peuvent être montées coulissantes ou pivotantes deux parties 12, 13 formant capot, visibles sur la figure 6, adaptées à être ouvertes pour ouvrir et charger le dispositif. En variante, une seule partie de capot est aussi possible. Le corps 10 peut être de forme environ arrondie, mais il pourrait avoir toute autre forme appropriée. Le corps 10 comporte un embout buccal 11 définissant un orifice de distribution 15 à travers lequel l'utilisateur va inhaler lors de l'actionnement du dispositif.

A l'intérieur du corps 10, il est prévu une bande 20 de réservoirs individuels prédosés 21, également appelés blisters, réalisée sous la forme d'une bande souple allongée sur laquelle les blisters sont disposés les uns derrière les autres, de manière connue. Cette bande de blisters 20 est du type pelable avec une couche de base 22 comportant les cavités formant les blisters et une couche de fermeture 23 pelable qui se décolle progressivement de la couche de base 22 à chaque actionnement.

Avant la première utilisation, la bande de blister 20 peut être enroulée à l'intérieur du corps 10, de préférence dans une partie de stockage, et des moyens de déplacement de bande 30 sont prévus pour progressivement dérouler et faire avancer cette bande de blister 20. La partie de couche de base 22 comportant les cavités vides est avantageusement adaptée à s'enrouler dans un autre endroit dudit corps 10, notamment une première partie de réception, et la partie de couche de fermeture décollée 23 est avantageusement adaptée à s'enrouler dans encore un autre endroit dudit corps 10, notamment une seconde partie de réception.

La première partie de réception comporte avantageusement un premier élément rotatif de réception 40, autour duquel la partie de couche de base 22 comportant les cavités vides s'enroule. Avantageusement, ce premier élément de réception rotatif 40 est sollicité en rotation par un ressort (non représenté), notamment un ressort à spirale disposé à l'intérieur dudit premier élément de rotation. Ceci garantit un bon enroulement à chaque actionnement.

La seconde partie de réception comporte avantageusement un second élément rotatif de réception 50, autour duquel la partie de couche de fermeture décollée 23 s'enroule. Avantageusement, ce second élément de réception rotatif 50 est également sollicité en rotation, notamment en étant corrélé en rotation avec les moyens de déplacement 30 de la bande de blisters 20. Ceci garantit un bon enroulement à chaque actionnement.

Les moyens de déplacement 30 sont adaptés à faire avancer la bande de blisters 20 avant et/ou pendant chaque actionnement du dispositif. De préférence, les moyens de déplacement comportent une roue d'indexage 30 qui reçoit et guide les blisters. Une rotation de cette roue d'indexage 30 fait avancer la bande de blister 20. Avantageusement, cette roue d'indexage 30 est tournée lorsque le dispositif est chargé, par exemple par l'ouverture du capot.

Selon un aspect avantageux, l'inhalateur comporte une chambre de dispersion 70 qui est destinée à recevoir la dose de poudre après ouverture d'un réservoir respectif. Cette chambre de dispersion 70 comporte une entrée 710, directement reliée au réservoir ouvert par un canal d'amenée de poudre 60, et une sortie 720, directement reliée à l'orifice de distribution 15 par un canal de distribution de poudre 80. La chambre de dispersion 70 contient de préférence au moins une bille 71, avantageusement plusieurs billes, par exemple trois. Ces billes 71 peuvent se déplacer dans ladite chambre de dispersion 70 le long d'un chemin de bille 75 sensiblement circulaire qui s'étend dans un plan environ transversal à l'orientation des canaux d'amenée 60 et de distribution 80. Ainsi, lesdits canaux d'amenée et de distribution s'étendent dans la même direction sensiblement perpendiculaire audit plan transversal dudit chemin de bille. La chambre de dispersion 70 comporte au moins une, de préférences deux entrées d'air 72. Ces entrées d'air 72 sont formées de préférence par des canaux d'air 90 qui débouchent dans la chambre de dispersion 70 de manière sensiblement tangentielle. Ainsi, ces entrées d'air tangentielles sont disposées dans ledit plan transversal dudit chemin de bille. Elles sont donc ainsi sensiblement perpendiculaires auxdits canaux d'amenée et de distribution. De cette manière, les flux d'air entrant par ces canaux d'air 90 vont tourbillonner dans la chambre de dispersion 70 et entrainer les billes 71 en rotation sur le chemin de bille 75. Ces flux d'air, qui sont crées par l'inhalation de l'utilisateur vont se mélanger au flux d'air et de poudre qui vient depuis le réservoir ouvert à travers le canal d'amenée 60, et qui pénètre dans la chambre de dispersion 70 par l'entrée 710. Ce flux d'air et de poudre va alors être également entraîné en rotation dans la chambre de dispersion 70, et les billes 71 vont désagglomérer la poudre avant que celle-ci ne sorte de la chambre de dispersion 70 par ladite sortie 720, d'où elle va être transférée à l'orifice de distribution 15 par ledit canal de distribution 80. Dans la position représentée sur la figure 1, les canaux d'amenée 60 et de distribution 80 s'étendent dans la même direction, qui est la direction verticale sur la figure 1, et le chemin de bille 75 et les canaux d'air 90 s'étendent dans un plan sensiblement horizontal dans la position de la figure 1.

Dans le mode de réalisation des figures 2 et 3, le canal d'amenée 60 est sensiblement coaxial avec le canal de distribution 80, les deux canaux débouchant dans la chambre de dispersion 70 sensiblement en son centre. Pour éviter que le flux d'air et de poudre venant du réservoir ouvert ne s'écoule directement dans le canal de distribution 80, ladite entrée 710 de la chambre de dispersion 70 comporte un déflecteur 100 qui dévie ledit flux d'air et de poudre en direction dudit chemin de bille 75 de la chambre de dispersion 70. Ce déflecteur 100 peut par exemple être formé par une partie de paroi courbe en forme de coupelle dont le sommet extérieur fait face au flux d'air et de poudre entrant. Une forme plus angulaire est aussi envisageable en variante à la forme arrondie représentée, et d'autres formes plus complexes de déflecteurs sont également possibles, l'essentiel étant de dévier le flux d'air et de poudre vers la chambre de dispersion 70 et ainsi d'éviter qu'il ne s'écoule en ligne droite directement dans le canal de distribution 80.

Selon un autre mode de réalisation avantageux, représenté sur les figures 5 et 7, le canal de distribution 80 s'étend de manière similaire au premier mode de réalisation, c'est à dire sensiblement verticalement à partir du centre de la chambre de dispersion 70 (dans la position de la figure 1). Par contre, le canal d'amenée 60 est décalé par rapport au canal de distribution 80, contrairement au premier mode de réalisation où ils étaient alignés. Ainsi, comme visible notamment sur la figure 5, le canal d'amenée 60 débouche directement dans le chemin de bille 75 de la chambre de dispersion 70. L'entrée 710 comporte donc un profil 715, tel qu'une grille, pour empêcher les billes 71 de tomber dans ledit canal d'amenée 60. Dans cette variante, un déflecteur n'est plus nécessaire, puisque le flux d'air et de poudre débouche directement dans le chemin de bille 75 où il se fait entraîner en rotation par le flux d'air et par les billes qui tournent. Néanmoins, on peut envisager quand même un déflecteur pour diriger au moins partiellement le flux d'air et de poudre vers la partie extérieure du chemin de bille, si nécessaire.

Lorsque le dispositif est actionné, la roue d'indexage 30 est tournée, ce qui amène le prochain blister 21 face à l'entrée 61 du canal d'amenée 60. Simultanément, la couche de couverture 23 est retirée de ce blister 21, en étant pelée par la rotation du second élément de réception rotatif 50 qui est avantageusement corrélée à la rotation de la roue d'indexage 30. Avantageusement, la couche de fermeture s'étend autour d'un coin de pelage 18 fixe qui, associé à la rotation simultanée de la roue d'indexage 30 et du second élément de réception rotatif 50 va provoquer le décollement ou pelage de la couche de fermeture 23 de la couche de base 22 au niveau dudit coin de pelage 18. La couche de base 22 en aval de la roue d'indexage 30 va s'enrouler autour du premier élément de réception rotatif 40. Ainsi, un blister ouvert 21 se retrouve face au canal d'amenée 60. Comme visible notamment sur les figures 2, 3, 5 et 7, les blisters 21 s'étendent transversalement par rapport à la bande de blisters 20. De cette manière, lorsque le blister ouvert se présente face à l'entrée 61 du canal d'amenée 60, cette entrée 61 s'étend sur environ la moitié de la longueur du blister, et l'autre moitié de la longueur se trouve face à la sortie 96 d'un canal d'arrivée d'air 95. Ainsi, lorsque l'utilisateur inhale, il va créer un premier flux d'air à travers ledit canal d'arrivée d'air 95. Ce premier flux d'air va traverser le blister ouvert 21, comme illustré par la flèche A1 sur les figures 2 et 5, et ainsi entraîner la poudre sous la forme d'un flux d'air et de poudre dans le canal d'amenée 60, comme illustré par les flèches B. Simultanément, l'inhalation va créer des flux d'air secondaires qui vont pénétrer tangentiellement dans la chambre de dispersion 70 à travers les canaux d'air tangentiels 90, comme illustré sur les figures 4 et 8 par les flèches A2. Ces flux d'air secondaires vont faire tourner les billes 71 dans la chambre de dispersion 70. Lorsque le flux d'air et de poudre rencontre ces flux d'air secondaires et les billes en mouvement dans la chambre de dispersion 70, il va se créer des tourbillonnements illustrés par les flèches C sur les figures 2 et 5. Ceci va fluidifier et désagglomérer la poudre, qui finalement va s'échapper en direction de l'orifice de distribution 15 comme illustré par les flèches D sur les figures 2 et 5.

Les dimensions et orientations des différents canaux peuvent bien entendu être optimisées selon les spécificités de l'inhalateur, pour maximiser les performances de celui-ci.

Diverses modifications sont possibles pour un homme du métier sans sortir du cadre de la présente invention tel que défini par les revendications annexées.

## Revendications

1. Dispositif d'inhalation de poudre, comportant un corps (10) pourvu d'un orifice de distribution (15), une pluralité de réservoirs prédosés (21) contenant chacun une dose de poudre à distribuer, et des moyens d'ouverture de réservoir (30; 50; 18) pour ouvrir un réservoir (21) à chaque actionnement, le dispositif comportant une chambre de dispersion (70) comportant une entrée (710) reliée lors de l'inhalation à un réservoir ouvert (21) et recevant le flux d'air et de poudre à partir dudit réservoir ouvert via un canal d'amenée (60), et une sortie (720) reliée audit orifice de distribution (15) via un canal de distribution (80), ladite chambre de dispersion (70) comportant au moins une bille (71) déplaçable sur un chemin de bille (75) dans ladite chambre de dispersion, ladite chambre de dispersion (70) comportant au moins une entrée d'air (72) environ tangentielle par rapport audit chemin de bille (75), **caractérisé en ce que** lesdits canaux d'amenée (60) et de distribution (80) s'étendent dans une même direction sensiblement perpendiculaire audit chemin de bille (75) et à ladite au moins une entré d'air tangentielle (72).

2. Dispositif selon la revendication 1, dans lequel ledit canal d'amenée (60) est sensiblement coaxial avec ledit canal de distribution (80), lesdites entrée (710) et sortie (720) de la chambre de dispersion (70) étant disposées sensiblement au centre de ladite chambre de dispersion.

3. Dispositif selon la revendication 2, dans lequel ladite entrée (710) de la chambre de dispersion (70) comporte un déflecteur (100) pour dévier le flux d'air et de poudre vers le chemin de bille (75) de ladite chambre de dispersion (70).

4. Dispositif selon la revendication 3, dans lequel ledit déflecteur (100) est en forme de coupelle avec le sommet extérieur de ladite coupelle tourné face au flux d'air et de poudre entrant.

5. Dispositif selon la revendication 1, dans lequel ledit canal d'amenée (60) est décalé par rapport audit canal de distribution (80), ladite entrée (710) de la chambre de dispersion (70) étant disposée au niveau du chemin de bille (75) et ladite sortie (720) de la chambre de dispersion (70) étant sensiblement au centre de ladite chambre de dispersion.

6. Dispositif selon la revendication 5, dans lequel ladite entrée (710) de la chambre de dispersion (70) comporte une grille.

7. Dispositif selon l'une quelconque des revendications précédentes, dans lequel chaque entré d'air (72) de la chambre de dispersion (70) est formée par un canal d'air tangentiel (90).

8. Dispositif selon l'une quelconque des revendications précédentes, dans lequel ladite pluralité de réservoirs (21) est formée sur une bande de blisters (20) souple allongée, comportant une couche de base (22) contenant les cavités des réservoirs et une couche de fermeture (23) recouvrant lesdites cavités.

9. Dispositif selon la revendications 8, dans lequel la couche de fermeture (23) est pelable de la couche de base (22), la partie de couche de base (22) contenant les blisters vides s'enroulant autour d'un premier élément de réception rotatif (40) et la partie de couche de fermeture (23) pelée de ladite couche de base s'enroulant autour d'un second élément de réception rotatif (50).

10. Dispositif selon la revendication 9, dans lequel lesdits moyens d'ouverture de réservoirs comportent des moyens pour décoller ladite couche de fermeture (23) de ladite couche de base (22), tels qu'un bord de pelage (18) autour duquel s'étend ladite couche de fermeture (23), le dispositif comportant des moyens de déplacement (30), tel qu'une roue d'indexage, pour faire avancer la bande de blisters (20) avant et/ou pendant chaque actionnement, l'extrémité aval de ladite couche de fermeture (23) étant fixée à un second élément de réception rotatif (50) dont la rotation est corrélée à la rotation de ladite roue d'indexage (30).

11. Dispositif selon l'une quelconque des revendications précédentes, dans lequel ladite chambre de dispersion (70) contient une pluralité de billes (71).

12. Dispositif selon l'une quelconque des revendications précédentes, dans lequel, après ouverture, ledit réservoir ouvert (21) se trouve disposé face d'une part audit canal d'amenée (60) et face à un canal d'arrivée d'air (95), de sorte que lors de l'inhalation, un flux d'air va pénétrer dans ledit blister (21) à travers ledit canal d'arrivée d'air (95) et entraîner ladite poudre dans un flux d'air et de poudre via ledit canal d'amenée (60) vers ladite chambre de dispersion (70).

## Patentansprüche

1. Pulverinhalationsvorrichtung, umfassend einen mit einer Ausgabeöffnung (15) versehenen Körper (10), mehrere vordosierte Behälter (21), die jeweils eine auszugebende Pulverdosis enthalten, und Behälteröffnungsmittel (30; 50; 18) zum Öffnen eines Behälters (21) bei jeder Betätigung, wobei die Vorrichtung eine Verteilungskammer (70) umfasst, die einen Einlass (710), der während der Inhalation mit einem geöffneten Behälter (21) verbunden ist und über einen Zufuhrkanal (60) den Luft- und Pulverstrom aus dem geöffneten Behälter empfängt, und einen Auslass (720) umfasst, der über einen Ausgabekanal (80) mit der Ausgabeöffnung (15) verbunden ist, wobei die Verteilungskammer (70) mindestens eine Kugel (71) umfasst, die auf einem Kugelpfad (75) in der Verteilungskammer verschiebbar ist, wobei die Verteilungskammer (70) mindestens einen Lufteinlass (72) in etwa tangential in Bezug auf den Kugelpfad (75) umfasst, **dadurch gekennzeichnet, dass** sich die Zufuhrkanäle (60) und Ausgabekanäle (80) in einer gleichen Richtung im Wesentlichen senkrecht zum Kugelpfad (75) und zum mindestens einen tangentialen Lufteinlass (72) erstrecken.

2. Vorrichtung nach Anspruch 1, wobei der Zufuhrkanal (60) im Wesentlichen koaxial zum Ausgabekanal (80) ist, wobei der Einlass (710) und der Auslass (720) der Verteilungskammer (70) im Wesentlichen in der Mitte der Verteilungskammer angeordnet sind.

3. Vorrichtung nach Anspruch 2, wobei der Einlass (710) der Verteilungskammer (70) einen Deflektor (100) umfasst, um den Luft- und Pulverstrom in Richtung zum Kugelpfad (75) der Verteilungskammer (70) abzulenken.

4. Vorrichtung nach Anspruch 3, wobei der Deflektor (100) in Form eines Schälchens vorliegt, wobei der äußere Scheitel des Schälchens zum eintretenden Luft- und Pulverstrom weist.

5. Vorrichtung nach Anspruch 1, wobei der Zufuhrkanal (60) in Bezug auf den Ausgabekanal (80) versetzt ist, wobei der Einlass (710) der Verteilungskammer (70) an dem Kugelpfad (75) angeordnet ist und der Auslass (720) der Verteilungskammer (70) im Wesentlichen in der Mitte der Verteilungskammer liegt.

6. Vorrichtung nach Anspruch 5, wobei der Einlass (710) der Verteilungskammer (70) ein Gitter umfasst.

7. Vorrichtung nach einem der vorhergehenden Ansprüche, wobei jeder Lufteinlass (72) der Verteilungskammer (70) durch einen tangentialen Luftkanal (90) gebildet ist.

8. Vorrichtung nach einem der vorhergehenden Ansprüche, wobei die mehreren Behälter (21) auf einem flexiblen, langgestreckten Blisterstreifen (20) gebildet sind, der eine Grundschicht (22), die die Hohlräume der Behälter enthält, und eine Verschlussschicht (23), die die Hohlräume bedeckt, umfasst.

9. Vorrichtung nach Anspruch 8, wobei die Verschlussschicht (23) von der Grundschicht (22) abziehbar ist, wobei sich der Teil der Grundschicht (22), der die leeren Blister enthält, um ein erstes drehbares Aufnahmeelement (40) aufrollt und sich der Teil der Verschlussschicht (23), der von der Grundschicht abgezogen wird, um ein zweites drehbares Aufnahmeelement (50) aufrollt.

10. Vorrichtung nach Anspruch 9, wobei die Behälteröffnungsmittel Mittel enthalten, um die Verschlussschicht (23) von der Grundschicht (22) zu lösen, wie eine Abziehkante (18), um welche sich die Verschlussschicht (23) erstreckt, wobei die Vorrichtung Verschiebemittel (30), wie ein Schaltrad, umfasst, um den Blisterstreifen (20) vor und/oder während jeder Betätigung voranzutreiben, wobei das nachgelagerte Ende der Verschlussschicht (23) an einem zweiten drehbaren Aufnahmeelement (50) befestigt ist, dessen Drehung der Drehung des Schaltrads (30) entspricht.

11. Vorrichtung nach einem der vorhergehenden Ansprüche, wobei die Verteilungskammer (70) mehrere Kugeln (71) enthält.

12. Vorrichtung nach einem der vorhergehenden Ansprüche, wobei der geöffnete Behälter (21) nach dem Öffnen einerseits gegenüber dem Zufuhrkanal (60) und gegenüber einem Lufteingangskanal (95) angeordnet ist, so dass bei der Inhalation ein Luftstrom über den Lufteingangskanal (95) in den Blister (21) eindringt und das Pulver in einem Luft- und Pulverstrom über den Zufuhrkanal (60) zur Verteilungskammer (70) mitnimmt.

## Claims

1. A powder inhaler device comprising: a body (10) that is provided with a dispenser orifice (15); a plurality of predosed reservoirs (21) each containing a dose of powder for dispensing; and reservoir-opening means (30; 50; 18) for opening a reservoir (21) on each actuation; the device further comprising a dispersion chamber (70) including an inlet (710) connected during inhalation to an open reservoir (21) and receiving the flow of air and of powder from said open reservoir via a delivery channel (60), and an outlet (720) connected to said dispenser orifice (15) via a dispenser channel (80), said dispersion chamber (70) including at least one ball (71) that is movable along a ball path (75) in said dispersion chamber, said dispersion chamber (70) including at least one air inlet (72) that is approximately tangential to said ball path (75), said device being **characterized in that** said delivery and dispenser channels (60 and 80) extend in a same direction that is substantially perpendicular to said ball path (75) and to said at least one tangential air inlet (72).

2. A device according to claim 1, wherein said delivery channel (60) is substantially coaxial with said dispenser channel (80), said inlet (710) and outlet (720) of the dispersion chamber (70) being arranged substantially at the center of said dispersion chamber.

3. A device according to claim 2, wherein said inlet (710) of the dispersion chamber (70) includes a deflector (100) so as to deflect the flow of air and of powder towards the ball path (75) of said dispersion chamber (70).

4. A device according to claim 3, wherein said deflector (100) is dish shaped with the outer tip of said dish facing the incoming flow of air and of powder.

5. A device according to claim 1, wherein said delivery channel (60) is offset relative to said dispenser channel (80), said inlet (710) of the dispersion chamber (70) being arranged at the ball path (75), and said outlet (720) of the dispersion chamber (70) being substantially at the center of said dispersion chamber.

6. A device according to claim 5, wherein said inlet (710) of the dispersion chamber (70) includes a mesh.

7. A device according to either preceding claim, wherein each air inlet (72) of the dispersion chamber (70) is formed by a tangential air channel (90).

8. A device according to either preceding claim, wherein said plurality of reservoirs (21) is formed on an elongate flexible blister strip (20) comprising a base layer (22) containing the cavities of the reservoirs, and a closure layer (23) overlying said cavities.

9. A device according to claim 8, wherein the closure layer (23) is peelable off the base layer (22), the portion of base layer (22) containing the empty blisters rolling up around a first rotary receiver element (40), and the portion of closure layer (23) peeled off said base layer rolling up around a second rotary receiver element (50).

10. A device according to claim 9, wherein said reservoir opening means include means for unsticking said closure layer (23) from said base layer (22), such as a peeling edge (18) around which said closure layer (23) extends, the device including displacement means (30), such as an indexer wheel, so as to cause the blister strip (20) to advance before and/or during each actuation, the downstream end of said closure layer (23) being fastened to a second rotary receiver element (50) that rotates in correlation with said indexer wheel (30).

11. A device according to either preceding claim, wherein said dispersion chamber (70) contains a plurality of balls (71).

12. A device according to either preceding claim, wherein, after opening, said open reservoir (21) is situated facing both said delivery channel (60) and an air delivery channel (95), such that during inhalation, a flow of air penetrates into said blister (21) through said air delivery channel (95) and entrains said powder in a flow of air and of powder via said delivery channel (60) towards said dispersion chamber (70).
